(19) Europäisches Patentamt
European Patent Office
Office européen des brevets

(11) **EP 2 878 314 B1**

(12) **EUROPEAN PATENT SPECIFICATION**

(45) Date of publication and mention of the grant of the patent:
**29.11.2017 Bulletin 2017/48**

(21) Application number: **13783091.5**

(22) Date of filing: **29.07.2013**

(51) Int Cl.:
*A61L 27/18* (2006.01)   *A61L 27/56* (2006.01)
*A61L 27/58* (2006.01)

(86) International application number:
**PCT/IB2013/056218**

(87) International publication number:
**WO 2014/016816 (30.01.2014 Gazette 2014/05)**

(54) **POLYMERIC MESH WITH SELECTIVE PERMEABILITY, FOR THE REPAIR AND REGENERATION OF TISSUES**

MALHA POLÍMERICA COM PERMEABILIDADE SELETIVA, PARA A REGENERAÇÂO/REPARAÇÃO DE TECIDOS

MAILLE POLYMÈRE À PERMÉABILITÉ SÉLECTIVE POUR LA RÉGÉNÉRATION/RÉPARATION DE TISSUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.07.2012 PT 10646412**

(43) Date of publication of application:
**03.06.2015 Bulletin 2015/23**

(73) Proprietor: **Association For The Advancement Of Tissue Engineering And Cell Based Technologies & Therapies (A4TEC)**
**4710-057 Braga (PT)**

(72) Inventors:
 • **PEREIRA MARTINS, Albino Manuel**
   **4820-005 Fafe (PT)**
 • **SOUSA DIAS ALVES DA SILVA, Marta Luísa**
   **4715-352 Braga (PT)**
 • **GONÇALVES DOS REIS, Rui Luís**
   **4250-242 Porto (PT)**
 • **MELEIRO ALVES DAS NEVES, Nuno João**
   **4700-207 Braga (PT)**

(74) Representative: **Silvestre de Almeida Ferreira, Luís Humberto**
   **Patentree, Lda.**
   **Edifício Net**
   **Rua de Salazares 842**
   **4149-002 Porto (PT)**

(56) References cited:
   **WO-A1-2011/003422    WO-A2-2009/099570**
   **JP-A- 2009 101 062**

 • **NEVES S C ET AL: "Chitosan/Poly(@?-caprolactone) blend scaffolds for cartilage repair", BIOMATERIALS, ELSEVIER SCIENCE PUBLISHERS BV., BARKING, GB, vol. 32, no. 4, 1 February 2011 (2011-02-01), pages 1068-1079, XP027514836, ISSN: 0142-9612, DOI: 10.1016/J.BIOMATERIALS.2010.09.073 [retrieved on 2010-10-27]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

# Description

## TECHNICAL FIELD

The present application describes a semi-porous elastic and biocompatible mesh with slow degradation and selective permeability, which can be used as a membrane or an implantable biomedical device. This mesh may be applied over the tissue defect in combination with routine surgical procedures such as the microfracture technique, promoting the repair and regeneration of tissues such as cartilage, skin, esophagus mucosa, and other tissues damaged by trauma or disease, in humans or other animals.

## STATE OF THE ART

The goal of tissue engineering is to design new functional components that can regenerate living tissue, restoring its function completely. The tissue engineering includes three basic premises: the use of a three-dimensional polymeric structure or scaffold that supports the cell growth and the subsequent formation of a tissue, a source of cells and growth factors that induce these cells to differentiate into the target tissue. These scaffolds can be developed for various purposes such as adhesion and fixation of tissues, maintenance of shape as guides for tissue regeneration, or as temporary support for their development. The biodegradable materials have been widely used in tissue engineering due to their capability to be degraded and metabolized by the body, after the repair of the damaged tissue. Thus, as the cells grow and organize into a living tissue, the scaffold is degraded slowly, because it is no longer needed as supporting structure. Therefore, the degradation degree of a scaffold is very important in the context of tissue engineering.

An implantable scaffold should recap the most essential characteristics of the native extracellular matrix of the tissue to be repaired, promoting the ideal conditions for the regeneration of the same. Thus, a very important feature of the scaffold is its porosity. High levels of porosity are desirable to maximize the opportunities for cells colonization which allow a faster tissue development. However, high levels of porosity involve less mechanical stability of the scaffold. Thus, an optimum balance between these conflicting requirements must be obtained for each specific application. The size of the pores is another critical issue in the cellular performance of scaffolds because they must have pores that allow cell adhesion and proliferation and, at the same time, permit the passage of nutrients and molecules present in the medium. Specifically, if the pores are too small, they can prevent the internal colonization of the scaffolds by the cells. In fact, this is a common limitation of meshes or membranes produced by electrospinning, which can be overcome by applying certain strategies proposed in the literature.

The majority of the processing methods do not allow specifying the size, the shape or the spatial distribution of pores. Often, these methods allow a tight control over the raw materials to be used and may allow establishing stable processing routines, which results in scaffolds with similar levels of porosity and macroscopic properties. Most methods may also allow obtaining some control over the interconnectivity level of the pores. However, the possible non-uniform distribution of the interconnectivity implies that some parts of the scaffold may not be colonized by the cells. Specifically, low interconnectivity levels hinder the local diffusion of fluids and the hydrolysis of biomaterials, resulting in irregular degradation kinetics.

The scaffolds can have several shapes, one of which is the mesh or membrane. Currently, there are many kinds of membranes available on the market for tissue regeneration, including cartilage, esophagus, periodontal ligament or skin. Most of these membranes are made of natural polymers and, consequently, they are biodegradable. They provide a solution for the regeneration of some tissue defects, but a complete regeneration and recovery of those tissues function has not been reported in the literature yet.

The regeneration of articular cartilage trauma or defects is still a major challenge in the field of orthopaedic medicine. Its avascular nature causes a very low innate capability for self-repair and regeneration. A wide variety of pathological conditions involve loss of articular cartilage such as osteoarthritis.

The existing strategies to repair or restore articular cartilage include total or partial joint arthroplasty, arthroscopic debridment, microfracture and tissue engineering approaches. Joint arthroplasty, or the total replacement of the joint, is one of the most successful interventions for cartilage related diseases such as osteoarthritis, but has several drawbacks, such as long term pain. Moreover, knee arthroscopy does not alter the osteoarthritis progression and its use leads to several undesired long term pain, cause by prosthesis instability or loosening of its components, or arthrofibrosis. Arthroscopic debridment and washing allow the removal of degenerative cartilage by irrigating the joint with a lactate and salt solution. This method leads to a temporary pain relief, but does not contribute significantly to new cartilage formation. Microfracture is a one-step arthroscopic procedure, involving a debridment and exposure of the subchondral bone, followed by a piercing of the bone. This results in bleeding from the bone marrow and a clot formation when the blood and bone marrow infiltrate in the damaged cartilage, releasing stem cells for the repair and regeneration of the tissue. Just like the arthroscopic debridment, the microfracture procedure leads to fibrocartilage tissue formation that will degenerate with time, though it can provide temporary symptom relieve.

In the last decade, tissue engineering (TE) approaches have reached the clinic, by combining cells and scaffolds. The general concept is that the scaffold structure will support cells growth and differentiation with full regeneration of the tissue. In recent years, cartilage TE has been expanded to OA diseased joint cartilage. The first approach for cartilage TE has been termed autologous chondrocyte transplantation (ACT), and has become the dominant clinical cell based therapy for the repair of cartilage lesions over the past decade. In this technique, expanded articular chondrocytes are implanted under a periosteal flap after surgical debridement of the lesion. ACT has demonstrated excellent short to mid-term repair although the evaluation of long-term repair remains somewhat controversial. The technique has suffered some improvements and the use of scaffolds as support for cell growth: MACI - Matrix Induced Chondrocyte Implantation. The scaffolds function is to support cell colonization, growth and differentiation, generating cellularized medical implants for substitution or regeneration of the damaged tissue. In the MACI technique, other sources of autologous cells besides chondrocytes may be used. Those sources include MSCs, periosteum-derived cells and synovial fibroblasts. A report of a 5 years follow-up of MACI technology showed good results: 8 out of 11 patients rated the function of their knees as much better or better than before the surgery. However, this approach involves two surgical interventions, as well as several drawbacks concerning the patient mobility and quality of life.

WO 2011/003422 A1 describes a biodegradable scaffold for soft tissue regeneration. The scaffold comprises a PLGA mesh having pores with a size in the range of 30-200 micrometers, a tensile strength superior than 1 MPa, an elongation at break in the range of 20-1000%, degrading in 4-36 months.

WO 2009/099570 A2 describes electrospinning PCL/ collagen nanofiber meshes for skeletal muscle regeneration.

JP 2009 101062 A describes a fiber assembly comprising chitosan fibers having a mean fiber diameter of 20 nm to 2 micrometers, a mean pore size of 0.05-50 micrometers and a thickness for the non-woven fabric of 50-200 micrometers.

NEVES S C ET AL: "Chitosan/Poly($\varepsilon$-caprolactone) blend scaffolds for cartilage repair", BIOMATERIALS, ELSEVIER SCIENCE PUBLISHERS BV., BARKING, GB, vol. 32, no. 4, 1 February 2011, pages 1068-1079, ISSN: 01.42-9612, describes a chitosan-PCL blend fibrous meshes for cartilage repair, having a porosity of 64.3%-83.2%, with a mean pore size between 265.3-384.7 micrometers.

## BRIEF DESCRIPTION

The present application describes an implantable mesh for the repair and regeneration of human or other animal tissue as for example articular cartilage, esophagus, periodontal ligament, skin, among others, damaged by trauma or disease. The semi-porous mesh described in the present invention is resistant to rupture, is elastic, biocompatible and of slow degradation, can be combined with the microfracture surgical procedure and allowing a fast tissue formation. This new mesh allows the confinement to a certain place of the cells from the tissue to repair and regenerate, these cells slow development and that the newly formed tissue presents similar characteristic to the pre-existent one

The tissue to be repaired by the polymeric mesh for tissue regeneration can be from cartilage, from periodontal ligament, from esophageal submucosa or skin.

In tissue regeneration, namely in the articular cartilage of the knee, the bone marrow stem cells, exposed to the lesion site by the surgical technique of microfracture, will be responsible by the restorative and regenerative processes of the damaged articular cartilage, by the spatial confinement conferred by the mesh described in the present invention in the form of membrane or biomedical device described in the present application that is a semi-porous elastic biocompatible mesh with slow degradation.

The mesh described in the present application is porous, the pores have smaller dimensions than the size of a human or other animal blood cell and allows, at the same time, the selective passage of biologically active molecules, coming from the bone marrow, from the synovial fluid or from the membrane itself. The use of the mesh herein described may also prevent, among others, the progression of traumatic lesions or of other initial forms of osteoarthritis, which in other case would lead to severe osteoarthritis, with persistent pain and loss of joint function, leading to the probable joint resection and substitution.

The present application describes a polymeric mesh for the repair and regeneration of tissues of an organism that comprises:

- Pores, wherein at least 70% of the pores of the said mesh have a size smaller than the one required to confine the cells of the said tissues, and wherein at least 70% of the pores of the said mesh has a size superior than the one needed for the passage of interstitial fluids of the said tissues; where preferentially 80% of the pores of the said mesh have a size smaller than the one required to confine the cells of the said tissues, most preferably 90%, 95% or 100%;

- the degradation time of the said polymeric mesh within the organism is at least 8 weeks, preferably the degradation time may vary between 8-26 weeks, and more preferably 8-15 weeks;

- the said polymeric mesh has an apparent elastic modulus between 0.1-100 MPa, preferably between 5-30 MPa;

- and the said polymeric mesh has an apparent tensile strength superior than 1 MPa, preferably the apparent tensile strength ranges between 2-30 MPa, preferably between 3-20 MPa.

The presence of pores in the mesh, whose dimensions are inferior to the size of a human or other animal cell, allow the bidirectional passage of biologically active molecules, for example the ones from the bone marrow, from the sinovial fluid or from other interstitial fluid. This mesh allows that the "new tissue" formed presents very similar properties to the ones of the damaged tissue.

The synergies of these characteristics of the mesh allow that the newly formed tissue presents very similar characteristics to the ones of the pre-existent tissue.

In one embodiment of the described polymeric mesh for the repair and regeneration of tissues, the bone marrow stem cells exposed to the lesion site by the microfracture surgical technique, will be confined to the cartilage defect by the fixation of the biomedical device, which consists in a biocompatible semi-porous elastic mesh with slow degradation rate, to the surface of the surrounding normal cartilage. The presence of pores in that mesh, which dimensions should be inferior to the size of a human or other animal cell, will allow bidirectional passage of biologically active molecules from the bone marrow, the synovial fluid or even from the mesh itself. Additionally, the implantable biomedical device could function as a local delivery system of biologically active molecules, which controls the inflammatory or regenerative processes, and/or the cartilage homeostasis.

In another embodiment of the described polymeric mesh for tissue regeneration has improved results with the following characteristics

- a mean porosity ranging between 83-91%, which can be determined by several commonly known methods, for example by the gravimetric method;

- 80-90% of the pores have a size ranging between 1.2-13.37 $\mu$m, which can be determined by several methods commonly known, for example by the mathematical model developed by Tomadakis and Robertson (Chemical Physics 2003, volume 119);

- an apparent elastic module ranging between 8.58-27.87 MPa; and an apparent tensile strength ranging between 2.15-4.38 MPa, which can be determined by several methods commonly known for example in traction tests, using for example a cell with a 1 kN charge and a temperature of 25 ° C.

These mechanic properties allow that the mesh for tissue regeneration presents better results in permeating the liquids surrounding the tissue to repair, in resisting to the forces exerted and in the confinement of the "regenerative" cells to the injury site, leading to the formation of a tissue with similar characteristics to the pre-existent one. These characteristics are relevant for example in its clinical application for articular cartilage regeneration, because it is intended that this mesh will separate the two compartments of the knee. It will separate the bone marrow cells from the synovial fluid, which will allow the chondrogenic differentiation of the bone marrow stem cells.

In one embodiment the referred polymeric mesh for the tissue regeneration described may comprise one polymer selected from the following list polycaprolactone, polyglycolic acid, polylactic acid, chitosan, alginate, dextran, or their combinations, these polymers can improve the tissue repair and regeneration. The use of these polymers allow supporting cells for a greater period of time, in order to allow them to form a denser extracellular matrix, giving a higher opportunity to the intended tissue formation, for example to the neo-cartilage formation, and therefore, to the defect repair.

In another embodiment the referred polymeric mesh for the tissue regeneration described may comprise a natural polymer, particularly a polymer selected from starch, hyaluronic acid, chondroitin sulfate or their combinations, this combination acts synergistically in the tissue repair, allowing a larger cell proliferation. In particular, the combination between the following polymers: polycaprolactone, or polycaprolactone and starch, or polycaprolactone and hyaluronic acid, or polycaprolactone and chondroitin sulfate, or polyglycolic acid, or polylactic acid, or their combination, between others.

In another preferential embodiment the described polymeric mesh for tissue repair and regeneration may comprise 60-80% (p/v) polycaprolactone and 20-40% (p/v) of a natural polymer, or any other proportion as for example 70% (p/v) polycaprolactone and 30% (p/v) of a natural polymer, in which the polycaprolactone weight may range between 70000 - 90000 Da.

In another preferential embodiment the described polymeric mesh for tissue repair and regeneration may comprise, where the deacetylation degree of chitosan ranges preferably between 50-100%, preferably between 87.7-95%.

This mesh allows confining the cells to the damaged area of the tissue to repair, and it is permeable to growth factors that enhance cell communication, without letting cells "escape" from the intended area allowing for the effective tissue regeneration, similar to a non-injured part of the tissue.

These mechanic properties allow that the mesh for tissue regeneration presents better results in permeating the liquids surrounding the tissue to repair, in resisting to the forces exerted and in the confinement of the "regenerative" cells to the injury site, leading to the formation of a tissue with similar characteristics to the pre-existent one. These characteristics are relevant for example in its clinical application for articular cartilage regeneration, because it is intended that this mesh will separate the two compartments of the knee. It will separate the bone marrow cells from the synovial fluid, which will allow the chondrogenic differentiation of the bone marrow stem cells.

In another embodiment the described polymeric mesh for tissue regeneration may further comprises active substances selected from the following list: anti-inflammatory drugs, antibiotics, biological agents, biological disease-modifying antirheumatic drugs, inhibitors, growth factors. The incorporation of active substances may be performed by mixture with the polymer(s) used to produce the mesh, by physic confinement, or by coating and/ or adsorption to the mesh surface.

The referred mesh may also act as a local delivery system of biologically active molecules, which controls the inflammatory or regenerative processes, and/or the cartilage homeostasis. In another embodiment the described polymeric mesh for the tissue regeneration may further comprises active substances involved in the homeostasis and the tissue regeneration process, in particular cartilage as for example TGF-$\alpha$, TGF-$\beta$1, TGF-$\beta$2,TGF-$\beta$3, EGF, HIF, PDGF-AA, PDGF-AB, PDGF-BB, PDGF-CC, PDGF-DD, VEGF-A, VEGF-B, VEGF-C e VEGF-D, IGF-1, FGF-2, FGF-18, BMP-2, BMP-4, BMP-6,BMP-7/OP-1, CDMP-1/GDF-5, CDMP-2 or equivalents.

The polymeric mesh for the tissue regeneration described may present a thickness that ranges between 20-200 $\mu$m, preferably 40-80 $\mu$m, and preferably 60 $\mu$m.

The present application also describes the use of the polymeric mesh for tissue regeneration in human and veterinary medicine, namely in the treatment of diseases involving tissue regeneration.

The polymeric mesh for tissue regeneration previously described may be applied in humans or animals, by fixation in the normal tissue surrounding the damaged area by the preferential use of a linker, such as a sealant glue or equivalent, or by a normal minimal invasive surgery, or by open surgery.

The polymeric mesh for tissue regeneration previously described may be applied in the regeneration of skin, cartilage, periodontal ligament, or esophageal submucosa.

The described polymeric mesh can be obtained by the electrospinning technique, although other processing techniques may be used, as long as the obtained polymeric meshes obey to the requisites previously established. Therefore, the mesh previously described can be woven or nonwoven.

In another preferential embodiment the strip, net, fibers bundle, mesh or membrane obtained by electrospinning comprising a set of fibers whose polymeric composition may be selected from the following list polycaprolactone, polyglycolic acid, polylactic acid, chitosan, alginate or dextran, or combinations thereof, with a diameter inferior to 2 $\mu$m.

In another embodiment the described strip, net, fibers bundle, mesh or membrane may contain a polymeric fiber obtained by electrospinning with a diameter between 0.35-2 $\mu$m.

In another embodiment the polymeric fiber of the strip, net, fibers bundle, mesh or membrane the molecular weight of polycaprolactone may range between 70000-90000 Da.

The fibers and meshes described previously may be obtained by electrospinning, particularly when the polymer is selected from the following list polycaprolactone, polyglycolic acid, polylactic acid, chitosan, alginate or dextran, or their combinations, with a diameter inferior to 2 $\mu$m, the following steps may be performed:

- Preparation of a solution of the selected polymer with a n appropriate solvent mixture; for example mixing a 13-17% (w/v) polycaprolactone solution, with a mixture of the solvents cloroform:dimetrilformamide in the ratio of 1:1-7:3
- Ejecting the resulting solution of the previous step with a flow rate ranging between 0.9-1 ml/h; the applied tension may vary between 9-12 kV; at a temperature that can range between 19-22,5 °C; a relative humidity that can range between 32-43 %.

## DETAILED DESCRIPTION

The present application describes a polymeric semi-porous biodegradable mesh, which can be used concurrently with the preferred form of embodiment of a surgical method file, allowing obtaining better results for the patient.

In another embodiment the polymeric mesh for tissue repair and regeneration can be used together with a surgical procedure, particularly the microfracture. This method has been employed in the clinic for tissue repair, namely damaged articular cartilage, using the organism self-regeneration ability. To produce a viable route for the marrow elements, the bony bed should be prepared adequately. The subchondral bone must be fully exposed and the base of the defect debrided of any soft tissue, particularly any remaining fibrous tissue. The calcified cartilage layer should be removed from the surface of the subchondral bone with an arthroscopic shaver or curette to enhance the defect repair and regeneration. The subchondral plate should be as completely preserved as possible. Secondly, the boundary of the defect needs to be shouldered by intact articular cartilage. The perimeter of the defect should ideally be perpendicular to the subchondral plate.

After the described preparation, the microfracture of the subchondral plate is performed with tapered awls and gentle mallet taps. The perforations provide channels for the marrow elements to migrate into the defect. These perforations should be made perpendicular to the subchondral bone, therefore the use of awls rather than drill bits is advised. Additionally, drilling may also cause thermal necrosis of the subchondral bone.

The microfracture is firstly performed at the periphery of the defect with the holes penetrating approximately 3 to 4 mm into the subchondral bone. The holes at the periphery may provide optimal anchoring of healing fibrocartilage to the transitional zone between normal articular cartilage. Perforations are then distributed uniformly throughout the defect with 3 to 4 mm bridges of subchondral bone between them. If the holes are placed too close together, the fractures may coalesce together and propagate at the subchondral bone. The tourniquet is released and inflow pressure reduced to confirm visualization of blood or fat droplets or both from all the microfracture holes. If this is not the case, the microfracture can be repeated to ensure marrow extrusion.

The microfracture releases blood and bone marrow from the medullar cavity that crosses the subchondral bone plate. In a first stage, a blood clot is formed at the microfracture sites. The stem cells and growth factors from the bone marrow transform this clot into a scar tissue named fibrocartilage.

The non-confinement of the blood clot to the cartilage lesion site induces its release into the synovial fluid, decreasing the treatment efficiency. Additionally, this blood clot is extremely fragile and has to be protected from physiological mechanic efforts. This problem is solved by applying a semi-porous polymeric mesh that allows the repair and regeneration of the tissue, this mesh could be used as a medical device/ biodegradable membrane.

The polymeric mesh for the repair and regeneration of tissues from an organism that comprises:

- Pores, wherein at least 70% of the pores of the said mesh have a size smaller than the one required to confine the cells of the said tissues, and wherein at least 70% of the pores of the said mesh has a size superior than the one needed for the passage of interstitial fluids of the said tissues; preferably, 80% of the pores of the said mesh have a size smaller than the one required to confine the cells of the said tissues, most preferably 90%, 95% or 100% of the pores; the pore size can be determined by several methods commonly known as for example by the mathematical model developed by Tomadakis and Robertson (Chemical Physics 2003, volume 119);

- where the degradation time of the said polymeric mesh within the organism is at least 8 weeks, preferably the degradation time may vary between 8-26 weeks, and preferably 8-15 weeks;

- the polymeric mesh has an apparent elasticity module ranging between 0.1 - 100 MPa, preferably between 5-30 MPa, which can be determined by several methods commonly known for example in traction tests, using for example a cell with a 1kN charge and a temperature of 25 °C;

- and the referred polymeric mesh and the said polymeric mesh has an apparent tensile strength superior than 1 MPa, preferably the apparent tensile strength ranges between 2-30 MPa, preferably between 3-20, which can be determined by several methods commonly known for example in traction tests, using for example a cell with a 1 kN charge and a temperature of 25 °C.

In this sense, the polymeric mesh for tissue repair and regeneration described in this application has slow degradation rate, and is semi-porous, allowing for the selective circulation of biologically active molecules between the blood clot that is confined to the cartilage lesion volume and the synovial cavity, or vice-versa. This can be explained by the pore dimension that should be sufficiently inferior to the minimal dimension of human or other animals cells, acting as a barrier to the passage of blood and bone marrow coming from the induced microfractures, by the polymer having a *in vivo* degradation time higher than 8 weeks, by the polymeric mesh having also sufficient mechanical stability to resists rupture (to resist for example to surgical manipulation, during the implantation and fixation processes), as well as having the sufficient elasticity to support physiological mechanic efforts existing in the lesion site (for example in the joint). The synergies of these mesh characteristics allow that the newly formed tissue presents characteristics very similar to the ones of the pre-existent tissue.

In another embodiment the described polymeric mesh for tissue regeneration has improved results with the following characteristics

- a medium porosity ranging between 83-91 %, which can be determined by several commonly known methods, for example by the gravimetric method;

- 80-90% of the pores have a size ranging between 1.2-13.37 μm, which can be determined by several methods commonly known, for example by the mathematical model developed by Tomadakis and Robertson (Chemical Physics 2003, volume 119);

- an apparent elasticity module ranging between 8.58-27.87 MPa; and an apparent tensile strength ranging between 2.15-4.38 MPa, which can be determined by several methods commonly known for example in traction tests, using for example a cell with a 1 kN charge and a temperature of 25 °C.

The implantable biomedical device in the form of biocompatible slow degradating mesh should present sufficient mechanical stability to resist its surgical manipulation, during the implantation and fixation processes, as well as the sufficient elasticity to support physiological mechanic efforts existing in the joint.

In another embodiment, to obtain an improved conversion process of the tissue to regenerate, as for example the conversion of a blood clot into fibrocartilage; the polymeric mesh for the regeneration should have a slow *in vivo* degradation rate of at least 8 weeks, and the best results were obtained with the polymers selected from the following list:

- Synthetic polymers such as for example polycaprolactone (PCL), polyglycolic acid (PGA), polylactic acid (PLA), and their co-polymers;

• Natural polymers such as for example chitosan, alginate, dextran;

• Combinations of synthetic origin polymers with natural origin polymers,

which degradation is extended until the minimal necessary limit to the fibrocartilagineous tissue formation.

It could be further added other polymers such as one polymer selected between starch, hyaluronic acid, or chondroitin sulfate, or their combinations.

The described polymeric mesh can be obtained by electrospinning technique, although other materials processing techniques may be used, as long as the obtained polymeric meshes obey to the requisites previously established.

The present polymeric semi-porous biocompatible and slow degradating mesh may further incorporate biologically active molecules. This incorporation may be performed by mixture with the biomaterials used to produce the mesh, by physic confinement, or by coating and/ or adsorption to the mesh surface. As biologically active molecules are referred anti-inflammatory drugs, namely anti-inflammatory non-steroid drugs (NSAIDs) (i.e. acetylsalicylic acid, paracetamol, choline magnesium trisalicylate, diclofenac, diflunisal, fenoprofen, flurbiprofen, ibuprofen, indomethacin, ketoprofen, meclofenamate, nabumetone, naproxen, oxaprozin, phenylbutazone, piroxicam, salsalate, sulindac, tolmetin) and cyclo-oxigenase-2 inhibitors (such as celecobix), corticosteroids or glucocorticoids, the disease-modifying anti-rheumatic drugs (DMARDs) (i.e. methotrexate, sulfasalazine, leflunomide, cyclosporine, minocycline, hydroxychloroquine, azathioprine, D-penicillamine and gold salts), and the disease-modifying biological agents, such as the tumoral necrose inhibitor $\alpha$ (such as the etanercept, infliximab, adalimumab, certolizumab, golimumab), the cytokines inhibitors such as interleukine-1$\alpha$ and -1$\beta$ (for example, anakinra) and -6 (for example, tocilizumab), the T-cell co-stimulation inhibitors (for example, the abatacept) and antibodies against B cells (for example, rituximab). It can be also incorporated biologically active molecules involved in the homeostasis and the tissue regeneration process, in particular transforming growth factor -$\alpha$, -$\beta$1, -$\beta$2 and -$\beta$3 (TGF-$\alpha$, TGF-$\beta$1, TGF-$\beta$2 and TGF-$\beta$3), the platelet-derived growth factors -AA, -AB,-BB, -CC and -DD (PDGF-AA, PDGF-AB, PDGF-BB, PDGF-CC and PDGF-DD), insulin growth factor-1 (IGF-1), the vascular endothelial growth factor -A, -B, -C e -D (VEGF-A, VEGF-B, VEGF-C and VEGF-D), the hypoxic induction factor (HIF), the epidermal growth factor (EGF), fibroblastic growth factors -2 and -18 (FGF-2 and FGF-18), the bone morphogenic proteins -2, -4, -6 and - 7/osteogenic protein-1 (BMP-2, BMP-4, BMP-6 and BMP-7/OP-1), cartilage-derived morphogenic proteins-1/growth factor and differentiation -5 and -2 (CDMP-1/GDF-5 and CDMP-2). The release of those molecules from the mesh will induce biologic effects on the blood clot confined to the volume of the cartilage defect and on the synovial cavity.

The fixation of this implantable biomedical device/ polymeric mesh for repair and regeneration of tissues should be performed preferably by using a linker, namely glue or sealant. However, other surgical membranes fixation methods can be used, specifically the use of resorbable sutures, staples or pins.

All the surgical procedure described above, including the surgical technique of microfracture, implantation of the biomedical device in form of a membrane and its fixation in the cartilage area surrounding the defect can be performed by the standard minimally invasive surgical method named arthroscopy or by open surgery.

The post-surgery monitoring of the human or other animal articular cartilage repair and regeneration processes, when using the device described on this invention, may be performed by arthroscopic examination, X-ray imaging, nuclear magnetic resonance and computerized tomography, biochemical analysis of the synovial fluid or blood.

**Method to calculate the mechanical properties of the mesh - elastic modulus and tensile strength**

The mechanical properties of the mesh, such as the apparent elastic modulus, the apparent tensile strength and the strain at break, may be determined by commonly known methods such as the tensile test.

To determine the apparent elastic modulus, the apparent tensile strength and the strain at break of the mesh, the following steps were developed:

• six rectangular samples (30 x 6 mm) of each mesh were used and individually fixed in a paper frame (30 mm x 25 mm) with a 15 mm square working window.

• the determinations were conducted in a universal tensile testing machine [Series 5543, INSTRON] equipped with a 1 kN loading cell and a crosshead speed of 5 mm/min was defined. Each paper frame holding a mesh sample was initially clamped by wedge action grips and manually closed by knurled screws. The lateral sides of that paper frame were cut and the tensile test was initiated.

• all tests were made under controlled environmental conditions, namely at a room temperature of 25°C. The test was defined to finish when sample fracture was achieved. The testing data was collected by the software Bluehill 2, being it informed about the apparent dimensions of each specimen before running the test. The apparent elastic modulus was defined as the slope of the line between the points corresponding to the 1 % and 2% of strain from the stress-strain curves determined automatically. The apparent tensile strength was defined as the maximum tensile stress supported by the material during the tensile test. The strain at break refers to the maximum elongation observed at the material break.

**Method to calculate the porosity - Gravimetry**

The porosity ($\varepsilon$) was determined by gravimetry, for three samples of each fiber mesh, by applying the formula:

$$\varepsilon = 1 - \frac{\rho_{APP}}{\rho_{PCL}}$$

The PCL density ($\rho_{PCL}$) has the value of 1.145 g/mL at 25 °C, according to the raw material datasheet. The apparent sample density ($\rho_{APP}$) was measured as the mass to volume ratio:

$$\rho_{APP} = \frac{scaffold\ mass}{scaffold\ thickness\ \cdot\ disc\ area}$$

The samples were prepared by using a punch with an inner diameter of 20mm. All the tests were made under controlled environmental conditions, namely at a room temperature of 25°C. The mass of each sample was determined by a precison balance [TP-214, Denver Instruments], by the arithmetic mean of three separate weighings. The thickness of each sample was determined by a microcaliper [IP65, Mitutoyo] with a flat tip (06 mm) and an accuracy of $\pm 1 \mu m$, by the arithmetic mean of six measurements at random positions of the sample.

**Method to calculate the estimated pore size, fibers diameter and porosity**

The mean pore size was calculate based on the relationship between the characteristic pore size ($d_{3D}$), the fiber diameter ($\omega$) and the porosity ($\varepsilon$), as shown in the equation below, developed by Tomadakis and Robertson (Chemical Physiscs 2003, volume 119):

$$d_{3D} = -\frac{\omega}{\ln(\varepsilon)}$$

This model was derived from a computer generated random fibrous structure in which a characteristic sphere with a 3D pore diameter was fit in. The equation assumes a random structure composed of a single fiber whose cross sections assume perfect round shapes. The fibers diameter ($\omega$) was measured in five independent images of scanning electron microscopy at 10000X, through the software AxioVision LE v4.8.2.0.

**Results obtained *in vitro* to polycaprolactone fiber meshes, in the regeneration of cartilage**

Description of the *in vitro* results obtained with polycaprolactone fiber mesh envisioning a cartilage regeneration application.

The tested mesh comprises:

• a porous polymeric mesh prepared with polycaprolactone with a molecular weight ranging between 70000-90000 Da,
• obtained by electrospinning, with an average fiber diameter between 0.35-2.0 $\mu$m,
• thickness 40-70 $\mu$m;
• mean pore size 1.2-13.37 $\mu$m
• porosity 83-91%.
• apparent tensile strength: 2.15-4.38 MPa
• apparent elastic modulus: 8.58-27.87 MPa

Considering a dynamic cell culture, the capacity of the fibrous PCL meshes to withstand the forces exerted by the liquid perfusion (between 0.10 - 100 $\mu$l/min) grants them a unique characteristic. This mechanical characteristic will be important when implanted in the synovial cavity of the knee, as it will allow that the cellularized implant supports the forces exerted by the synovial fluid. Furthermore, the dynamic system culture highlights the permeability of these fibrous meshes to liquids/ fluids but not to cells, which gives then selective advantages in the exchange of soluble factors released by cells in culture. In being selective to these factors, the mesh will allow communication between cells, hindering their "escape" from the mesh. This characteristic will be relevant for its clinic application in articular cartilage regeneration, as we intend that this mesh acts as a division between the two compartments of the knee. The mesh will separate cells from the bone marrow from the liquid of the synovial cavity, which will allow the chondrogenic differentiation induction of the mesenchymal stem cells present in bone marrow.

In order to simulate the division performed by the mesh when implanted into the synovial cavity of the knee, we seeded human articular chondrocytes and human stem cells in distinct sides of the biodegradable fibrous mesh. Preliminary results showed that the chondrocytes cultured on one side of the mesh were able to induce the chondrogenic differentiation of the stem cells cultured on the opposite side of the fibrous biodegradable mesh, without adding bioactive agents for chondrogenic differentiation. It could also be observed that either chondrocytes or stem cells were not able to pass through the fibrous biodegradable mesh, specifically from one side to the other of the mesh.

The surface of these meshes may be altered by surface modification, aiming at improving their biocompatibility and surface bioactivity characteristics. In a recent work, chondroitin sulfate (CS), a natural origin polymer, was immobilized at the surface of the fibers from the PCL meshes, which were previously functionalized by UV/O$_3$ exposure and aminolysis. Contact angle, SEM, optical profilometry, FTIR, X-ray photoelectron spectroscopy techniques confirmed the success of CS-immobilization in PCL NFMs. Furthermore, CS-immobilized PCL NFMs showed lower roughness and higher hydrophilicity than the samples without CS. Human articular chondrocytes (hACs) were cultured on the PCL fibrous meshes with or without CS immobilization. Biological results showed that hACs proliferated through the entire time course of the experiment in both types of meshes tested. The deposition of cartilage extracellular matrix was also observed on the two types of biodegradable fibrous meshes. Quantitative-PCR results demonstrated over-expression of cartilage-related genes such as *Aggrecan, Collagen type II,* COMP and *Sox9* on both types of biodegradable fibrous meshes. Morphological observations from SEM and LSCM revealed that hACs maintained their characteristic round shape and cellular agglomeration exclusively on the PCL fibrous meshes with CS immobilization. In conclusion, CS immobilization at the surface of PCL fibrous meshes was achieved successfully and provides a valid platform enabling further surface functionalization methods in scaffolds to be developed for cartilage tissue engineering.

Another advantage of these polycaprolactone fibrous meshes concerns its biodegradation. Polycaprolactone has a slow degradation rate (between 8 - 15 week) when compared to commercially available meshes. This higher degradation time allows supporting cells longer, in a way that cells are able to produce a denser extracellular matrix, allowing for the neo-cartilage formation and consequently, the repair of the cartilage defect.

**Results obtained *in vitro* to polycaprolactone fiber meshes, in the regeneration of esophagus**

Description of the *in vitro* results obtained with polycaprolactone fiber meshes envisioning an esophagus tissue regeneration application.

The tested mesh comprises:

- a porous polymeric mesh prepared with polycaprolactone with a molecular weight ranging between 70000-90000 Da,
- obtained by electrospinning, with an average fiber diameter between 0.35-2.0 $\mu$m,
- thickness 40-70 $\mu$m;
- mean pore size 1.2-13.37 $\mu$m
- porosity 83-91%.
- apparent tensile strength: 2.15-4.38 MPa
- apparent elastic modulus: 8.58-27.87 MPa

A biodegradable fiber mesh was implanted in the animal model domestic pig, in an esophageal defect of 4 cm of circumferential diameter, induced by endoscopic submucosal dissection (ESD) of the esophagus, as a model of premalignant and low grade malignant esophageal lesions. This *in vivo* study demonstrated the ability of an implanted biodegradable fiber mesh to prevent the occurrence of esophageal stenosis after ESD. Fourteen days after implantation of the biodegradable fiber mesh into the defective esophagus, an endoscopic assessment showed macroscopic signs of re-epithelialization of the esophageal submucosa. The implanted biodegradable fiber mesh, besides allowing the culture of epithelial cells, also prevents the bleeding and the perforation of the esophagus. In addition, the biodegradable polycaprolactone fiber mesh exhibited resistance to enzymatic degradation of the luminal contents from the esophagus. The results of this preclinical study with animal survival indicate that the implantable biodegradable fiber mesh allows re-epithelialization and remodeling of the esophageal mucosa with less fibrosis formation, avoiding luminal diameter reduction of the esophagus.

Technically, the biodegradable fiber mesh is easy to handle, considering the limitations of handling endoscopic equipment, with the necessary flexibility and other physical properties essential for endoscopic application. Upon implantation, the biodegradable fiber mesh showed good adaptability to the gastrointestinal tissues, with high guarantee of placement in the defect area, maintaining this position throughout the regenerative period, not despising the constant presence of luminal secretions and contractility of the esophagus. Generally, the biodegradable fiber mesh presents structural sustainability that, theoretically, enable to isolate fragile areas of the wall of tubular organs such as the esophagus and the rectum.

**Results obtained *in vitro* to polycaprolactone fiber meshes, in the regeneration of periodontal ligament**

Description of the *in vitro* results obtained with polycaprolactone fiber meshes envisioning a periodontal ligament regeneration application.

The tested mesh comprises:

- a porous polymeric mesh prepared with polycaprolactone with a molecular weight ranging between 70000-90000 Da,
- obtained by electrospinning, with an average fiber diameter between 0.35-2.0 $\mu$m,
- thickness 40-70 $\mu$m;
- mean pore size 1.2-13.37 $\mu$m
- porosity 83-91%.
- apparent tensile strength: 2.15-4.38 MPa
- apparent elastic modulus: 8.58-27.87 MPa

The biological functionality of the biodegradable fiber mesh can be improved by culturing human periodontal ligament derived cells (hPDLCs) prior to implantation. Phenotypic characterization of the hPDLCs was conducted by flow cytometry. Research data demonstrated the expression of the typical mesenchymal stem cells (MSCs) antigens like CD-29, -73, -90 and -105, through 15 culturing passages, and the absente expression of hematopoietic markers, including CD-34 antigen and -45. Those MSCs were seeded on electrospun polycaprolactone nanofiber meshes (PCL NFM) and on BioGide® membranes (porcine collagen-based membranes from Geistlich Biomaterials) to ascertain the biological behavior of hPDLCs.

Experimental data showed similar biological performance (i.e. cell viability and proliferation, and protein synthesis) on both studied structures, under basal and osteogenic culture conditions. Interestingly, hPDLCs cultured on PCL NFM under basal conditions tend to present significant lower expression of osteoblastic markers, namely of alkaline phosphatase. This observation is pertinent given the fact that this condition favors the formation of a normal ligament and could prevent the ankilosis of the tissue, preventing the development on non-functional periodontal tissue. On the other hand, the expression of bone-related proteins and transcripts corroborates the successful osteogenic differentiation of hPDLCs when cultured on both PCL NFM and BioGide®.

In conclusion, the biodegradable fiber mesh is an integrated solution for the regeneration of periodontal ligament. This mesh allows, simultaneously, the local delivery of drugs and the culture of relevant cells, maximizing the effectiveness of the treatment and the recovery time of the patient. Technically, it is easier to apply and does not collapse during surgery.

**Results obtained *in vitro* to chitosan fiber meshes, as skin wound dressing**

Description of the *in vitro* results obtained with chitosan fiber meshes envisioning a skin wound dressing application.

Characterization of the mesh:

*   Polymeric composition: chitosan (molecular weight: 270-416 kDa; deacetilation degree (DD): 87.7-95%);
*   Average fibers diameter: 65 nm - 6 $\mu$m;
*   Non-woven mesh, composed by fibers randomly distributed or with preferred alignment directions;
*   Degradation: water absorption: ~300% after 7 days; weigth loss: ~30% after 7 days.

Description of the electrospinning method to obtain chitosan fiber meshes:

*   Polymer concentration: 5-6 % (w/v)
*   Solvents: trifluoroacetic acid: dichloromethane (7:3)
*   Flow rate: 0.4-0.8 ml/h
*   Voltage: 12-18 kV
*   Spinneret to ground collector distance: 10-12 cm
*   Spinneret internal diameter: 0.514 mm
*   Ambient temperature: 19-22.5 °C
*   Relative Humidity: 32-43 %

Description of the neutralization process:

The neutralization process prevents the dissolution of the mesh when immersed in aqueous media. The mesh is immersed in a solution of 7N ammonia in methanol during 10 minutes, washed with distilled water until neutral pH (pH = 7) and finally frozen at -180 °C. The water removal is done by freeze-drying to preserve the fiborus structure of the mesh.

Detailed Description of the Application

A biodegradable fiber mesh made of chitosan for the treatment of dermal wounds and burns and/or regeneration of skin. This mesh acts as a barrier to unwanted cells (such as bacteria) being permeable to nutrients and gases. It has an adequate physical integrity and adaptability to the dermal defect, displaying easy handling both in dry and wet state. It has the ability to absorb large amounts of water and also to release the water absorbed after adhering to the skin. Chitosan has functional groups (specifically amino groups, - $NH_2$), which allows the immobilization of bioactive agents and/or nanoparticles. Hence, the fiber mesh may include one or more drugs and/or one or more proteins and/or desirable cells, reducing the side effects of bioactive agents and the number of secondary surgical procedures.

The *in vitro* capacity of such chitosan fiber mesh to absorb and release antibiotics, particularly gentamicin, was assessed, as well as the release of gentamicin from nanoparticles (for example, liposomes) immobilized at the surface of fibers from the mesh. To achieve that, the chitosan fiber meshes were functionalized with thiol groups (-SH) and the liposomes covalently immobilized at the surface of the fibers, presenting an uniform distribution. The release kinetics study showed that gentamicin is released from the liposomes immobilized at the surface of the fiber mesh in a sustained manner over a period of 16 hours. The susceptibility tests showed that the gentamicin released from the chitosan fiber meshes, as well as form the liposomes immobilized at the surface of the fibers from the mesh, inhibits the growth of *Escherichia coli, Pseudomonas aeruginosa* and *Staphylococcus aureus.* The results show that these meshes can be used with success in local skin application, in eradicating pathogens commonly present in skin infections and/or dermal holes.

The present invention is not, in any way, restricted to the embodiments described herein and a person of ordinary skills in the area can provide many possibilities to modifications thereof without departing from the invention, which is defined in the claims.

The preferred embodiments described above are obviously combinable. The following claims define further preferred embodiments of the present invention.

**Claims**

1. A polymeric mesh for the repair and regeneration of tissues of an organism that comprises pores wherein at least 70% of the pores of the said mesh have a size smaller than the one required to confine the cells of the said tissues, and wherein at least 70% of the pores of the said mesh has a size more than the one needed for the passage of interstitial fluids of the said tissues;
   wherein the degradation time of the said polymeric mesh within the organism is at least 8 weeks and the said polymeric mesh comprises:

   an apparent tensile strength between 2.15-4.38 MPa and an apparent elastic modulus between 8.58-27.87 MPa;
   an interconnected porosity ranging between 83-91%;
   in which 80-90% of the pores have a size ranging between 1.2-13.37 $\mu$m.

2. A mesh, according to the previous claim, wherein the degradation time of the said polymeric mesh is 8-26 weeks, preferably 8-15 weeks.

3. A mesh, according to the previous claims, comprising at least one polymer selected from polycaprolactone, polyglycolic acid, polylactic acid, chitosan, alginate or dextran, or combinations thereof.

4. A mesh, according to the previous claim, further comprising a natural polymer selected from starch, hyaluronic acid, or chondroitin sulfate, or combinations thereof.

5. A mesh, according to claims 3-4, wherein the polymeric mesh consists in the following polymers:

   polycaprolactone, or
   polycaprolactone and starch, or
   polycaprolactone and hyaluronic acid, or
   polycaprolactone and chondroitin sulfate, or
   polyglycolic acid and polylactic acid, or combinations thereof.

6. A mesh, according to any one of the claims 3-5 wherein said mesh comprises 80-60% (w/v) of polycaprolactone and 20-40% (w/v) from a natural polymer, or any other polymers proportion.

7. A mesh, according to any one of the claims 3-6, wherein the molecular weight of polycaprolactone ranges between 70000 - 90000 Da.

8. A mesh, according to claims 3-4, wherein the molecular weight of chitosan ranges between 270-416 kDa.

9. A mesh, according to claims 3-4, wherein the deacetylation degree of chitosan ranges between 50-100%, preferably between 87.7-95%.

10. A mesh, according to the previous claims, further comprising active substances selected from the following list: anti-inflammatory drugs, antibiotics, biological agents, biological disease-modifying antirheumatic drugs, inhibitors, growth factors.

11. A mesh, according to the previous claims, comprising active substances selected from the following list: acetylsalicylic acid, paracetamol, choline magnesium trisalicylate, diclofenac, diflunisal, fenoprofen, flurbiprofen, ibuprofen, indomethacin, ketoprofen, meclofenamate, nabumetone, naproxen, oxaprozin, phenylbutazone, piroxicam, salsalate, sulindac, tolmetin, corticosteroids, glucocorticoids, methotrexate, sulfasalazine, leflunomide, cyclosporine, minocycline, hydroxychloroquine, azathioprine, D-penicillamine, etanercept., infliximab, adalimumab, certolizumab, golimumab, anakinra, tocilizumab, abatacept, TGF-$\alpha$, TGF-$\beta$1, TGF-$\beta$2, TGF-$\beta$3, EGF, HIF, PDGF-AA, PDGF-AB, PDGF-BB, PDGF-CC, PDGF-DD, VEGF-A, VEGF-B, VEGF-C e VEGF-D, IGF-1, FGF-2, FGF-18, BMP-2, BMP-4, BMP-6, BMP-7/OP-1, CDMP-1/GDF-5, or CDMP-2.

12. A mesh, according to the previous claims, wherein its thickness ranges between 20-200 $\mu$m, preferably 40-80 $\mu$m.

13. A mesh, according to the previous claim, for use in the treatment of diseases that involve the repair and regeneration of tissues, in particular tissue to be treated is skin, cartilage, periodontal ligament, or esophageal submucosa.

14. A mesh, according to the previous claim, wherein said mesh is woven or nonwoven.

15. Mesh according to any of the previous claims obtainable by electrospinning comprising a set of fibers containing a polymeric composition selected from the following list: polycaprolactone, polyglycolic acid, polylactic acid, chitosan, alginate or dextran, or combinations thereof, the fibers diameter being less than 2 $\mu$m.

**Patentansprüche**

1. Ein poröses Polymernetz für die Reparatur und Regeneration von Gewebe eines Organismus, wobei mindestens 70 % der Poren des besagten Netzes eine Größe haben, die kleiner ist, als die Größe, die zum Zurückhalten der Zellen des besagten Gewebes notwendig ist, und wobei mindesten 70 % der Poren des besagen Netzes eine Größe haben, die über der für den Durchfluss der Gewebeflüssigkeit des besagten Gewebes notwendigen Größe liegt; wobei die Abbauzeit des besagten Polymernetzs im Organismus mindestens 8 Wochen beträgt und das besagte Polymernetz:

eine Zugfestigkeit zwischen 2,15-4,38 MPa und ein Elastizitätsmodul zwischen 8,58-27,87 MPa aufweist;
eine zwischen 83-91 % liegende zusammenhängende Porosität aufweist;
und bei dem 80-90 % der Poren eine Größe zwischen 1,2-13,37 $\mu$m aufweisen.

2. Ein Netzs gemäß dem vorherigen Anspruch, wobei die Abbauzeit des besagten Polymernetzes 8-26 Wochen beträgt, vorzugsweise 8-15 Wochen.

3. Ein Netz gemäß den vorherigen Ansprüchen, das mindestens ein Polymer aus Polycaprolacton, Polyglykolsäure, Polylactide, Chitosan, Alginate oder Dextran oder Kombinationen aus diesen enthält.

4. Ein Netz umfasst gemäß dem vorherigen Anspruch außerdem ein natürliches Polymer aus Stärke, Hyaluronsäure oder Chondroitinsulfat oder Kombinationen aus diesen.

5. Ein Netz gemäß den Ansprüchen 3-4, wobei das Polymernetz aus folgenden Polymeren besteht:

Polycaprolacton, oder
Polycaprolacton und Stärke, oder
Polycaprolacton und Hyaluronsäure, oder
Polycaprolacton und Chondroitinsulfat, oder
Polyglykolsäure und Polylactide, oder einer Kombination aus diesen.

6. Ein Netz gemäß einem der Ansprüche 3-5, wobei besagtes Netz zu 80-60 % (w/v) aus Polycaprolacton und zu 20-40 % (w/v) aus einem natürlichen Polymer oder einem anderen Polymeranteil besteht.

7. Ein Netz gemäß einem der Ansprüche 3-6, wobei das Molekulargewicht des Polycaprolacton zwischen 70000 - 90000 Da liegt.

8. Ein Netz gemäß den Ansprüchen 3-4, wobei das Molekulargewicht des Chitosans zwischen 270-416 kDa liegt.

9. Ein Netz gemäß den Ansprüchen 3-4, wobei der Deacetylierungsgrad des Chitosans zwischen 50-100 %, vorzugsweise zwischen 87,7-95 % liegt.

10. Ein Netz gemäß den vorherigen Ansprüchen, das außerdem Wirkstoffe enthält, die aus folgender Liste ausgewählt wurden: entzündungshemmende Arzneimittel, Antibiotika, biologische Arbeitsstoffe, biologische krankheitsmodifizierende Antirheumatika, Hemmstoffe, Wachstumsfaktoren.

11. Ein Netz gemäß den vorherigen Ansprüchen, das Wirkstoffe enthält, die aus folgender Liste ausgewählt wurden: Acetylsalicylsäure, Paracetamol, Cholin-Magnesium-Trisalicylat, Diclofenac, Diflunisal, Fenoprofen, Flurbiprofen, Ibuprofen, Indometacin, Ketoprofen, Meclofenamat, Nabumeton, Naproxen, Oxaprozin, Phenylbutazon, Piroxicam, Salsalat, Sulindac, Tolmetin, Corticosteroide, Glucocorticoide, Methotrexat, Sulfasalazin, Leflunomid, Ciclosporin, Minocyclin, Hydroxychloroquin, Azathioprin, D-Penicillamin, Etanercept, Infliximab, Adalimumab, Certolizumab, Golimumab, Anakinra, Tocilizumab, Abatacept, TGF-a, TGF-pi, TGF-P2 ,TGF-P3, EGF, HIF, PDGF-AA, PDGF-AB, PDGF-BB, PDGF-CC, PDGF-DD, VEGF-A, VEGF-B, VEGF-C und VEGF-D, IGF-1, FGF- 2, FGF-18, BMP-2, BMP-4, BMP-6,BMP-7/OP-1, CDMP-1/GDF-5 oder CDMP-2.

12. Ein Netz gemäß den vorherigen Ansprüchen, wobei dessen Stärke zwischen 20-200 $\mu$m, vorzugsweise 40-80 $\mu$m liegt.

13. Ein Netz gemäß den vorherigen Ansprüchen zur Behandlung von Krankheiten, die die Reparatur und die Regeneration von Gewebe beinhalten, insbesondere bei der Behandlung von krankem Gewebe, Knorpel, Wurzelhaut oder der Submukosa der Speiseröhre.

14. Ein Netz gemäß dem vorherigen Anspruch, wobei besagtes Netz ein Maschennetz oder ein Vliesstoff ist.

15. Ein Netz gemäß einem der vorherigen Ansprüche, dass durch Elektrospinnen gewonnen wird, bestehend aus einer Reihe von Fasern mit einer Polymerzusammensetzung, die aus folgender Liste ausgewählt wurde: Polycaprolacton, Polyglykolsäure, Polylactide, Chitosan, Alginate oder Dextran oder Kombinationen aus diesen mit einem Faserdurchmesser von weniger als 2 $\mu$m.

**Revendications**

1. Une maille polymère pour la réparation et la régénération des tissus d'un organisme qui comprend des pores dans lequel au moins 70% des pores de ladit mailleont une taille inférieure à celle requise pour confiner les cellules desdits tissus, et dans lequel au moins 70% des pores de ladit mailleont une taille supérieure à celle requise pour le passage des fluides interstitiels desdits tissus ;

dans lequel le temps de dégradation de ladit maillepolymère au sein de l'organisme est d'au moins 8 semaines et ladit maillepolymère comprend :

une résistance à la traction apparente située entre 2,15-4,38 MPa et un module d'élasticité apparent situé entre 8,58-27,87 Mpa ;
une porosité interconnectée située entre 83-91% ;
dans lequel 80-90% des pores ont une taille située entre 1,2-13,37 $\mu$m.

2. Une maille, selon la revendication précédente, dans lequel le temps de dégradation de ladit maillepolymère est situé entre 8-26 semaines, de préférence 8-15 semaines.

3. Une maille, selon les revendications antérieures, comprenant au moins un polymère sélectionné à partir de polycaprolactone, d'acide polyglycolique, d'acide polylactique, de chitosan, d'alginate ou de dextran, une d'une combinaison de ceux-ci.

4. Une mailleselon la revendication précédente, comprenant également un polymère naturel, sélectionné à partir d'amidon, d'acide hyaluronique, ou de sulfate de chondroïtine, ou d'une combinaison de ceux-ci.

5. Une mailleselon les revendications 3-4, dans lequel la maillepolymère est constitué des polymères suivants :

polycaprolactone, ou
polycaprolactone et amidon, ou
polycaprolactone et acide hyaluronique, ou
polycaprolactone et sulfate de chondroïtine, ou
acide polyglicolique et polylactique, ou une combinaison de ceux-ci.

6. Une maille, selon l'une quelconque des revendications 3-5 dans lequel ladit maillecomprend 80-60% (w/v) de polycaprolactone et 20-40% (w/v) d'un polymère naturel, ou toute autre proportion de polymères.

7. Une mailleselon l'une quelconque des revendications 3-6, dans lequel le poids moléculaire de la polyprolactone se situe entre 70000-90000 Da.

8. Une maille, selon les revendications 3-4, dans lequel le poids moléculaire du chitosan se situe entre 270-416 kDa.

9. Une mailleselon les revendications 3-4, dans lequel le degré de désacétylation du chitosan se situe entre 50-100%, de préférence entre 87,7-95%.

10. Une mailleselon les revendications antérieures, comprenant également des substances actives sélectionnées à partir de la liste suivante : médicaments anti-inflamatoires, antibiotiques, agents biologiques, médicaments antirhumatismaux modificateurs de la maladie biologiques, inhibiteurs, facteurs de croissance.

11. Une maille, selon les revendications précédentes, comprenant des substances actives selectionnées à partir de la liste suivante : acide acétylsalicylique, paracétamol, trisalicylate de magnésium de choline, diclofénac, diflunisal, fénoprofène, flurbiprofène, ibuprofène, indométhacine, kétoprofène, méclofénamate, nabumétone, naproxène, oxaprozine, phénylbutazone, piroxicam, salsalate, sulindac, tolmétine, corticostéroïdes, glucocorticoïdes, méthotrexate, sulfasalazine, leflunomide, cyclosporine, minocycline, hydroxychloroquine, azathioprine, D-pénicillamine, étanercept, infliximab, adalimumab, certolizumab, golimumab, anakinra, tocilizumab, abatacept, TGF-$\alpha$, TGF-$\beta$1, TGF-$\beta$2, TGF-P3, EGF, HIF, PDGF-AA, PDGF-AB, PDGF-BB, PDGF-CC, PDGF-DD, VEGF-A, VEGF-B, VEGF-C et VEGF-D, IGF-1, FGF2, FGF-18, BMP-2, BMP-4, BMP-6,BMP-7/OP-1, CDMP-1/GDF-5, ou CDMP-2.

12. Une maille, selon la revendication précédente, dans lequel son épaisseur se situe entre 20-200 $\mu$m, de préférence 40-80 $\mu$m.

13. Une maille, selon la revendication précédente, pour une utilisation dans le traitement de maladies qui requièrent la réparation et la régénération de tissus, particulièrement si le tissu devant être traité est de la peau, du cartilage, du ligament péridentaire, ou de la sous-muqueuse oesophagienne.

14. Une maille, selon la revendication précédente, dans lequel ladit maille est tissé ou non-tissé.

15. Maille selon l'une quelconque des revendications antérieures pouvant être obtenu par électrofilage comprenant un ensemble de fibres contenant une compositon polymère sélectionnée à partir de la liste suivante : polycaprolactone, acide polyglycolique, acide polylactique, chitosan, alginate ou dextran, ou une combinaison de ceux-ci, le diamètre des fibres étant inférieur à 2 $\mu$m.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011003422 A1
- WO 2009099570 A2
- JP 2009101062 A

### Non-patent literature cited in the description

- Chitosan/Poly(ε-caprolactone) blend scaffolds for cartilage repair. **NEVES S C et al.** BIOMATERIALS. ELSEVIER SCIENCE PUBLISHERS BV, 01 February 2011, vol. 32, 1068-1079
- **TOMADAKIS ; ROBERTSON.** *Chemical Physics,* 2003, vol. 119
- **TOMADAKIS ; ROBERTSON.** *Chemical Physiscs,* 2003, vol. 119